# EUROPEAN PATENT SPECIFICATION

(11) **EP 4 029 951 B1**
(45) Date of publication and mention of the grant of the patent: **05.02.2025**
(21) Application number: 21215227.6
(22) Date of filing: 16.12.2021
(51) Int. Cl.: C12Q 1/6844, C12Q 1/6848, C12Q 1/6851, C12Q 1/6853, C12Q 1/686

(54) **METHODS AND PRIMERS FOR DETECTING DHA CANOLA NS-B50027-4**
VERFAHREN UND PRIMER ZUM NACHWEIS VON DHA-RAPS NS-B50027-4
MÉTHODES ET AMORCES POUR DÉTECTER LE DHA CANOLA NS-B50027-4

(30) Priority: 16.12.2020 US 202063126360 P
(43) Date of publication of application: 20.07.2022
(73) Proprietor: Nuseed Nutritional Australia Pty Ltd, Laverton North VIC 3026 (AU)
(72) Inventor: GAO, Wenxiang, Woodland, 95776 (US); BAI, Yonghe, Woodland, 95776 (US)
(74) Representative: Bryers Intellectual Property Ltd

(56) References cited:
- WO-A1-2020/055763
- US-A1- 2018 016 590
- US-A1- 2018 016 591

## Description

### SEQUENCE LISTING

This Specification contains DNA sequences identified in a Sequence Listing entitled "METHOD AND PRIMERS FOR DETECTING DHA CANOLA NS-50027-4," 5000 bytes, created December 13, 2021, submitted in ASCII format via EFS-Web,.

### BACKGROUND

Long chain omega-3 (LC-ω3) fatty acids such as docosahexaenoic acid (DHA) provide many health benefits, and can be included in human diets, e.g., by consuming algae-derived products or algae-eating fish. Alternative sources for LC-ω3 fatty acids are needed to satisfy increased human consumption needs. A genetically-modified canola line, DHA canola NS-B50027-4, accumulates a significant concentration of DHA in canola seed. There remains a need for efficient identification of NS-B50027-4, e.g., for plant stewardship purposes. WO 2020/055763 A1 provides compositions and methods to identify DHA canola NS-B50027-4 and progeny thereof.

### SUMMARY

The present embodiments provide methods and primers to identify DHA canola NS-B50027-4 event.

The present invention provides a qualitative gel based method of identifying the presence of a DHA canola NS-B50027-4 even or progeny thereof as defined in claim 1. The dependent claims set out features of certain embodiments of the present invention.

One embodiment provides an agarose gel electrophoresis-based method to qualitatively detect the presence (or absence) of the Nuseed DHA Canola NS-B50027-4 event. More specifically, two event-specific PCR assays targeting the T-DNA insertion junction sites in the canola genome can be used for adventitious presence testing, trait purity testing, and trait introgression, and to support DHA Canola NS-B50027-4 plant stewardship.

### BRIEF DESCRIPTION OF THE DRAWINGS

FIG. 1 is an image of a gel showing GeneRuler low range DNA ladder. More specifically, Thermo Scientific^{™} GeneRuler^{™} Low Range DNA Ladder, ready-to-use, contains a mix often chromatography-purified individual DNA fragments (in base pairs): 700, 500, 400, 300, 200, 150, 100, 75, 50, and 25.
FIG. 2 shows primer locations for the primers used as described herein to identify the event NS-B50027-4 transgenic junction on chromosome A05. Primers A05-216F and A05-216R, underlined with arrows indicating direction; transgenic insert DNA, bold; *Brassica* DNA, normal text. The (+) strand of this junction region is also provided as SEQ ID NO:7.
FIG. 3 shows primer locations (boxed) for primers A02-258F (SEQ ID NO:3) and A02-258R (SEQ ID NO:4), for the transgenic junction region on chromosome A02 (SEQ ID NO:8).
FIG. 4 is a gel image from a qualitative event-specific assay (A05-216) for a 216-bp amplicon of AV Jade (negative control), no template control (NTC) samples, six different DHA canola NS-B50027-4 spike levels and eight conventional Nuseed canola lines. The 216-bp amplicon was unique to DHA canola NS-B50027-4.
FIG. 5 is a gel image from a qualitative event-specific assay (A05-216) for a 216-bp amplicon of sixteen replicates of event positive 0.05% spike samples.
FIG. 6 is a gel image showing the absence of the A05-216 amplicon in six different commercial canola GMO events and the presence of the HMG206-amplicon (206-bp).
FIG. 7A is a gel image from the qualitative event-specific assay A05-216 with eight canola Certified Reference Materials (CRM; seven genetically-modified (GM) and one regular canola materials) from AOCS. The 216-bp amplicon was absent in all of the eight canola CRM.
FIG. 7B is a gel image from the qualitative event-specific assay A05-216 with seven soybean, seven maize and five cotton Certified Reference Materials (CRM; four genetically-modified (GM) and one regular cotton materials) from AOCS. The 216-bp amplicon was absent in all of the CRM tested.
FIG. 8 shows primer locations (boxed) for primers A05-200F (SEQ ID NO:9) and A05-200R (SEQ ID NO: 10) for the transgenic junction on chromosome A05 (SEQ ID NO:11) for assay A05-200.
FIG. 9 is a gel image for assay A05-200 on six different DHA GMO-canola NS-B50027-4 spike samples. The 200-bp amplicon was present in all six different DHA GMO-canola NS-B50027-4 spike samples. The 206-bp amplicon generated from the internal reference canola HMG gene was present in AV Jade and HMG-206 spike sample.
FIG. 10 is a gel image showing for assay A05-200 showing the 200-bp amplicon was consistently detectable in fifteen replicates of event positive 0.05% spike samples.
FIG. 11 is a gel image from assay A05-200 plus HMG 206 with six CRM canola events. The 206-bp amplicon was amplified as expectedly in six of the commercial canola events, the 258-bp amplicon, however, did not.
FIGS. 12A and 12B are gel images from assay A05-200 with twenty-five different commercial GMO events ordered from AOCS. The 200-bp amplicon was absent in all of the commercial GMO events tested including canola events, seven soybean events, seven maize events, and four cotton events.
FIG. 13 is a gel image from assay A05-200 tested with seven non-GM conventional canola varieties selected from Nuseed germplasm pool with various genetic backgrounds. Assay A05-200 failed to amplify any amplicon from these seven non-GM conventional canola varieties

### DETAILED DESCRIPTION

It should be understood that this invention is not limited to the particular methodology, protocols, and reagents, etc., described herein and as such may vary. The terminology used herein is for the purpose of describing particular embodiments only, and is not intended to limit the scope of the present invention, which is defined solely by the claims.

As used herein and in the claims, the singular forms "a," "an," and "the" include the plural reference unless the context clearly indicates otherwise. Throughout this specification, unless otherwise indicated, "comprise," "comprises" and "comprising" are used inclusively rather than exclusively, so that a stated integer or group of integers may include one or more other non-stated integers or groups of integers. The term "or" is inclusive unless modified, for example, by "either." Thus, unless context indicates otherwise, the word "or" means any one member of a particular list and also includes any combination of members of that list.

All values are approximate as there is some fluctuation in fatty acid composition due to environmental conditions. Values are typically expressed as percent by weight of total fatty acid, or percent weight of the total seed. Accordingly, other than in the operating examples, or where otherwise indicated, all numbers expressing quantities or reaction conditions used herein should be understood as modified in all instances by the term "about."

NS-B50027-4 DHA canola was generated, *inter alia*, via *Agrobacterium tumefaciens*-mediated transformation of canola cultivar AV Jade with a binary vector designated pJP3416_GA7-ModB. Vector pJP3416_GA7-ModB was designed specifically to convert oleic acid to DHA in seed, and contains expression cassettes for seven microalgae and yeast transgenes (abbreviated Micpu-Δ6D, Pyrco-Δ5E, Pavsa-Δ5D, Picpa-ω3D, Pavsa-Δ4D, Lackl-Δ12D and Pyrco-Δ6E) involved in the DHA biosynthesis pathway (and also includes a PAT gene for herbicide tolerance selection). Each transgene has its own expression cassette comprising seed-specific promoter, enhancer, and terminator. *See* U.S. Patents No. 10,563,218 and No. 10,570,405.

NS-B50027-4 DHA canola was characterized with vector-targeted sequencing, whole-genome sequencing and Polymerase Chain Reaction (PCR)-amplicon sequencing. Sequencing data indicated that the DHA canola contained no vector backbone, no binary vector bacterial selectable marker gene Neomycin Phosphotransferase II (NPT II) or any *A. tumefaciens* genome sequence. Sequencing information also indicated that DHA canola contained two T-DNA inserts, one on chromosome A02 and the other on chromosome AOS. The full genomic DNA sequences of the two T-DNA inserts were verified and the sequence of each T-DNA insert perfectly matched the reference of vector pJP3416_GA7-ModB. Both T-DNA inserts were required to accumulate the desired amount of DHA in seed oil.

More specifically, molecular characterization of the NS-B50027-4 canola indicated that it carried two transgenic inserts, one on chromosome A02 and the other on chromosome A05, and that both transgenic inserts were required to accumulate about 10% DHA in canola seed. *See* Shunxue Tang, et al., Molecular Characterization of Genetically Modified Canola NS-B50027-4 Producing High Percentage of Long-Chain Omega-3 (LC-ω3) Fatty Acids in Seed, Nuseed Americas (December 14, 2016).

The A02 T-DNA insertion is a partial insert, containing complete gene expression cassettes for genes Micpu-Δ6D, Pyrco-Δ5E, Pavsa-Δ5D and Picpa-ω3D but not for genes Pavsa-Δ4D, Lackl-Δ12D, Pyrco-Δ6E, nor PAT. The sequence of the A02 T-DNA insert otherwise matches the reference of vector pJP3416_GA7-ModB. The A02 T-DNA insert is located within the 3' UTR of a hypothetical protein gene on chromosome A02.

The A05 T-DNA insertion contains two T-DNA transgene sets from the binary vector that formed a palindromic structure in the right border-transgenes-left border: left border-transgenes-right border orientation. The sequence of the A05 T-DNA insert also matches the reference sequence in vector pJP3416_GA7-ModB. The A05 T-DNA insert is located within the second exon of a Pto-Interacting (PTI) gene on chromosome A05.

The present embodiments provide methods and primers for the relatively easy identification of the Nuseed DHA canola NS-B50027-4 event. More specifically, the present embodiments provides a qualitative detection method for determining the presence of DHA Canola (Event NS-B50027-4) in oilseeds DNA sample. The assay can be used for adventitious presence testing, trait purity testing, and trait introgression, and to support DHA Canola NS-B50027-4 regulatory submission and commercialization. *See also* WO 2020/055763.

Event-specific gel-based assay, targeting an insert in DHA canola NS-B50027-4 on chromosomes A05 and A02, have been successfully developed and validated. The HMG reference gene PCR profile was designed to confirm the PCR ability of DNA sample.

The Limit of Detection (LOD) of the event-specific assay is determined at least 0.05% NS-B50027-4 DNA to total DNA, or less than 50 genome copies. The specificity of the assay was validated by testing 27 CRM materials available from AOCS and 7 different Nuseed non- GM conventional oilseeds varieties.

### EXAMPLES

### Example 1. Gel electrophoresis-based A05-216 qualitative event-specific assay for detection of the transgenic event in DHA canola NS-B50027-4.

### DNA Extraction

DNA were extracted from seeds using CTAB DNA extraction method briefly described below:
Step 1: Grind 3000 seeds per sample completely and transfer powder to 50 mL Falcon tubes. Clean grinder thoroughly between samples to prevent cross contamination
Step 2: Add 30 mL of 1x CTAB Extraction Buffer (1% CTAB, 50 mM Tris-HCl pH 8.0, and 10 mM EDTA pH 8.0) and mix thoroughly by shaking and inverting the tube several times.
Step 3: Incubate the samples in 55-60°C (57.5°C optimal) water bath for 1 hr. Mix the samples every 10 min by lightly inverting the tubes. After the incubation, let the samples cool down to room temperature.
Step 4: Centrifuge the samples for 2 min at 3000g. Carefully remove as much as possible the top layer, which is oil, using pipet. The light brown middle layer contains DNA. Centrifuge the samples again for 10 min at 3000g.
Step 5: For each sample, transfer 900 uL of the middle layer to a 2 mL centrifuge tube, add 900 µL chloroform under a fume hood. Mix the samples vigorously for 5 min. Centrifuge the samples for 5 min at 13000g.
Step 6: Transfer 750 µL of the supernatant to a new 2.0 mL centrifuge tube, add 750 µL 1x CTAB Buffer and mix by inverting the tubes 10-15 times. Then let the samples rest at room temperature on bench for 5 min. Proceed to centrifuge the samples at 13000g for 7 min. Discard the supernatant.
Step 7: Add 250 µL 1M NaCl solution containing RNase A (final concentration 20 µg/mL) to each sample and mix by inverting 5-10 times. Incubate samples in 50°C for 1 hr. Gently invert the tubes every 10 min during incubation.
Step 8: Let the samples cool down to room temperature. Then, add 500ul -20°C 100% ethanol. Invert the tubes gently for about 5 min to precipitate DNA. Centrifuge the samples at 13000g for 5 min and discard the supernatant. Wash the DNA pellet with 500 µL 70% Ethanol at room temperature for at least 30 min.
Step 9: Centrifuge the tubes at 13000g for 5 min and discard the supernatant. Dry the sample tubes under the fume hood for about 30-60 min. Do not over dry DNA pellet. Add 100 µL of H₂O to each sample and suspend the DNA by pipetting. Add 1uL DNase-free RNase A (10 mg/mL) to the DNA solution and incubate at 50°C water bath or oven for 60 min. Let the samples sit at room temperature for about 30 min before checking the DNA quantity, e.g., with Qubit4 fluorometer from Invitrogen.

Store the DNA samples at 4°C for temporary storage up to a week or at -20°C for long term storage.

### Event Specific PCR Method

### Preparation of samples with different DHA NS-B50027-4 DNA level spikes

Prepare 20 ng/µl DNA solutions from DHA canola NS-B50027-4 DNA and negative control AV Jade before making up the following spike samples, as provided in Table 1. Six DHA canola NS-B50027-4 spike levels were made as described below, the genome copies number of reference gene HMG and DHA canola is listed in Table 2.

| Table 1. Concentrations of spike samples | | | |
|---|---|---|---|
| 1. | 50% | spike sample: | Mix equal volume of the DHA canola DNA solution (20 ng/µL) and the negative control AV Jade DNA solution (20 ng/µL). |
| 2. | 10% | spike sample: | Dilute 5 times of the 50% spike sample with DNA solutions from the AV Jade (20ng/µL) to make up the 10% spike sample. |
| 3. | 1% | spike sample: | Dilute 10 times of the 10% spike sample with DNA from AV Jade (20 ng/µL) to make up the 1% spike sample. |
| 4. | 0.1% | spike sample: | Dilute 10 times of the 1% spike sample with DNA from AV Jade (20 ng/µL) to make up the 0.1% spike sample. |
| 5. | 0.05% | spike sample: | Dilute 2 times of the 0.1% spike sample with DNA from AV Jade (20 ng/µL) to make up the 0.05% spike sample. |
| 6. | 0.025% | spike sample: | Dilute 2 times of the 0.05% spike sample with DNA from AV Jade (20 ng/µL) to make up the 0.025% spike sample. |

| Table 2. Six spike level samples with corresponding reference gene genome copy number, and the copy number for NS-B50027-4 | | | | | | |
|---|---|---|---|---|---|---|
| DHA canola dilution series | 50% | 10% | 1% | 0.1% | 0.05% | 0.025% |
| Total amount of DNA (ng) | 100 | 100 | 100 | 100 | 100 | 100 |
| Target taxon HMG copies | 86957 | 86957 | 86957 | 86957 | 86957 | 86957 |
| GM % (NS-B50027-4) | 50 | 10 | 1 | 0.1 | 0.05 | 0.025 |
| NS-B50027-4 oilseed rape GM copies | 43478 | 8695 | 869 | 87 | 43.4 | 21.7 |

### PCR Assembly:

Assemble all reaction (25 µL/reaction) components (Cat Log #: M0480L) on ice as illustrated in Table 3.

| Table 3. PCR reaction assembly | |
|---|---|
| Components | Volume (µL) |
| 5X OneTaq Standard Reaction Buffer (NEB) | 5.0 |
| 10 mM of each dNTPs | 0.5 |
| 2.5 µM event specific assay mixture (forward and reverse primers mixture) or Internal Reference Gene (HMG) forward and reverse primers mixture* | 2.0 |
| OneTaq DNA Polymerase (5 units/µL) (NEB) | 0.2 |
| Template DNA (20 ng/µL)** | 5.0 |
| Nuclease-free water | 12.3 |
| Total reaction | 25.0 |
| * This applies to three separate experiments with: 1) Forward and reverse primers mixture for detecting the A02 insert; 2) Forward and reverse primers mixture for detecting the A05 insert; and 3) Forward and reverse primers mixture for detecting the endogenous HMG gene. | |
| ** Ne positive control, negative control and non-template control in each of the three experiments. | |

### PCR Cycling Profile:

PCR was used for amplification with the following profile parameters:

| Table 4. PCR cycling profile | | | | |
|---|---|---|---|---|
| | Stage | Temperature | Time | Cycles |
| 1 | Initial denaturation | 94 °C | 60 sec | 1 |
| 2 | Denaturation | 94 °C | 30 sec | 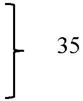 |
| 3 | Annealing | 58°C | 30 sec | |
| 4 | Elongation | 72°C | 30 sec | |
| 5 | Final extension | 72°C | 5 min | 1 |
| 6 | | 10°C | Forever | |

### Reference gene and DNA ladder

Internal reference gene: Canola HMG is used as the internal reference gene for this qualitative detection method. Primer sequences are provided in Table 5, below. The amplicon size for HMG is 206 bp.

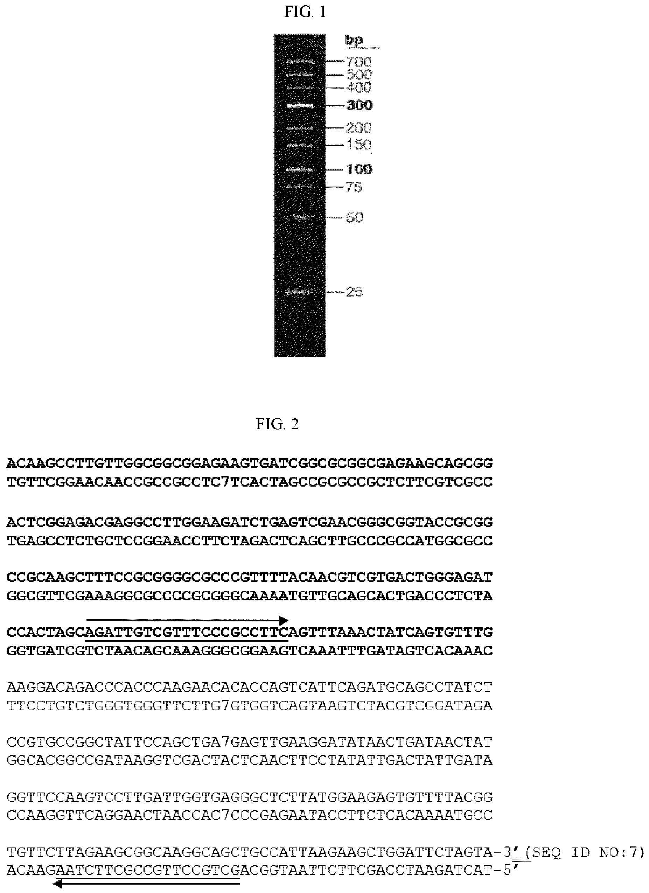

GeneRuler low range DNA ladder: Thermo Scientific^{™} GeneRuler^{™} Low Range DNA Ladder, ready-to-use, contains a mix often chromatography-purified individual DNA fragments (in base pairs): 700, 500, 400, 300, 200, 150, 100, 75, 50, and 25 (FIG. 1). Agarose gel electrophoresis

PCR products are resolved through agarose gel electrophoresis and analyzed with the Life Technology Image System. For each PCR reaction, 10 µL PCR products is mixed with 3 µL H2O and 1 µL loading dye (6x; Thermo Fisher Scientific Cat. Log# R1161) and run on a 2% agarose gel (gel size 14 cm long and 12 cm wide) at 100 Volts for 60 minutes. DNA ladder (6 µl per lane) is run on proper number of lanes to enable PCR amplicon sizes to be identified. Gel image is captured with the Life Technology Image system.

Chemicals and equipment that are needed for DNA extraction: CTAB, NaCl, Tris-HCl, EDTA, DNase-free RNase, Ethanol, Grinder, Centrifuge, Tubes, etc.

### Primers designed across the junctions of insertions in DHA canola

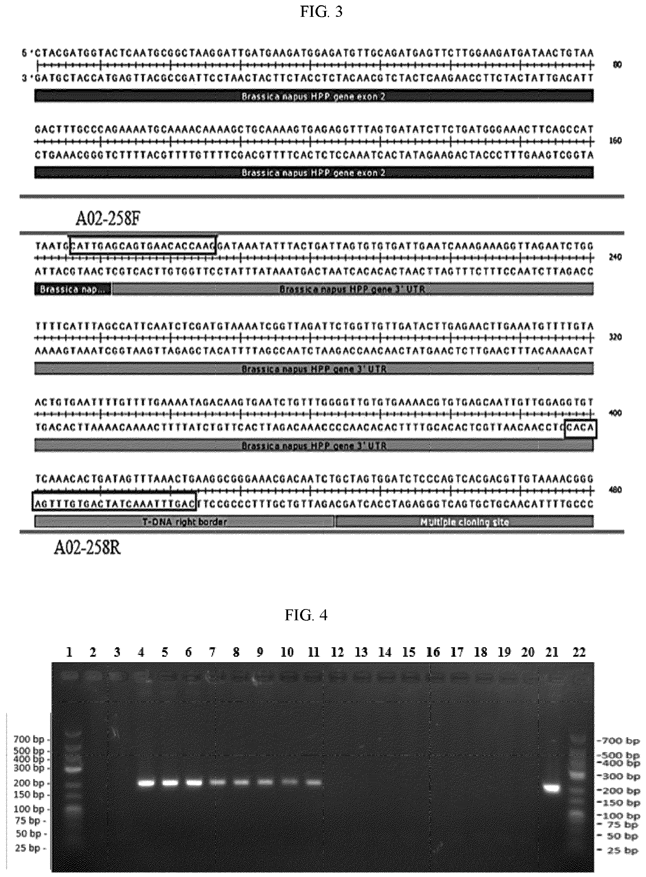

The qualitative event-specific assay (A05-216) was designed to detect the junction between T-DNA insert and genomic DNA on Chromosome A05. The qualitative event-specific assay (A02-258) was designed to detect the junction between T-DNA insert and genomic DNA on Chromosome A02, *see* WO/2020/055763. The primer sequences, locations, and product sizes are shown in Table 5 and FIGs. 2 and 3. The *Brassica* HMG gene was used as the reference gene for the internal control recommended by Chinese National Standard (MARA 2031-9-2013). Primer sequences and the expected amplicon sizes are listed in the following table. The primer pair A02-258F and A02-258R is used to detect the insert or junction on chromosome A02, and the amplicon size is 258 bp. The primer pair A05-216F and A05-216R is used to detect the insert or junction on chromosome A05, and the amplicon size is 216 bp. The primer pair Hmg206F and Hmg206R is used to detect the endogenous HMG gene (internal reference gene HMG as control), and the amplicon size is 206 bp.

| Table 5. Primer sequences for assay A05-216 and the reference gene HMG | | |
|---|---|---|
| Primer Name | Primer Sequence | Amplicon Size |
| A05-216F | AGATTGTCGTTTCCCGCCTTC (SEQ ID NO: 1) | 216 bp |
| A05-216R | GCTGCCTTGCCGCTTCTAA (SEQ ID NO:2) | |
| A02-258F | CATTGAGCAGTGAACACCAAG (SEQ ID NO:3) | 258 bp |
| A02-258R | CAGTTTAAACTATCAGTGTTTGAACAC (SEQ ID NO:4) | |
| Hmg206F | TCCTTCCGTTTCCTCGCC (SEQ ID NO:5) | 206 bp |
| Hmg206R | TTCCACGCCCTCTCCGCT (SEQ ID NO:6) | |

### Testing Samples:

A hundred nanogram (ng) genomic DNA template of the following samples were subject to event-specific qualitative PCR (Assay A05-216 for junction in chromosome A05 and Assay HMG for Reference Gene):
Event NS-50027-4 as positive control for event-specific Assay (A05-216);
Canola AV Jade as negative control for event-specific Assay (A05-216) and positive control for Reference Gene Assay HMG;
Seven Non-GM conventional canola varieties selected from Nuseed germplasm pool: NX0026, NX0331, NX0953, NX0980, NX1012, NX1302, NX1306;
Two Certified Non-Modified Reference Materials (Leaf DNA) purchased from American Oil Chemists' Society (AOCS): Canola 0306-B4, Cotton 0306-A4;
Twenty-Five Certified GM Reference Materials (Leaf DNA or seeds powder) purchased from AOCS:
   Seven Canola Events: Topas19/2, T45, Rf2, Rf1, Ms8, Ms1, Rf3;
   Seven Soybean Events: MON89788, MON87769, MON87708, MON87705, MON87701, FG72, A5547-127;
   Seven Maize Events: GA21, MON89034, MIR604, MON88017, MON87427, MON87460, T25; and
   Four Cotton Events: MON15985-7, MON531, MON1445, GHB614.

The results from the qualitative event-specific assay with 6 different DHA canola NS-B50027-4 spike levels.

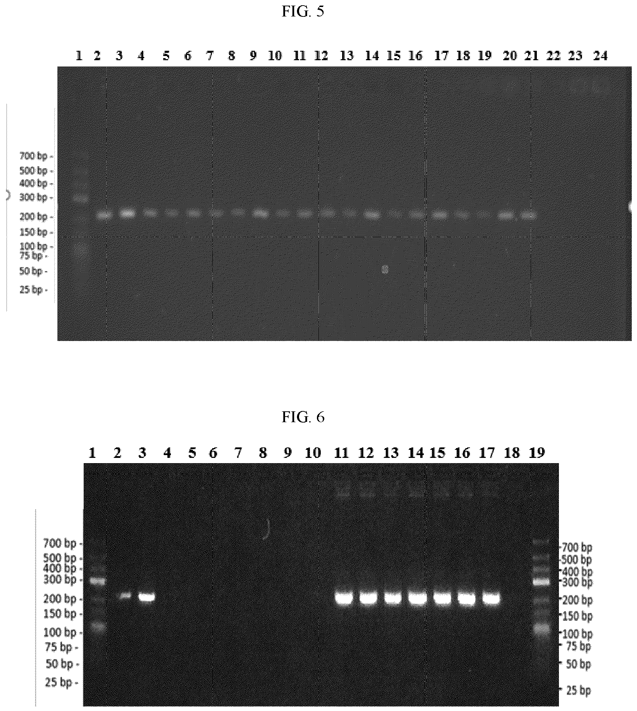

The qualitative event-specific assay (A05-216) was tested with AV Jade, NTC, six different DHA canola NS-B50027-4 spike levels, and eight conventional Nuseed canola lines (FIG. 4). The results from 16 replicates showed the assay can consistently detect the expected amplicons at least 0.05% spike level (FIG. 5). Gel lanes for FIG. 4 and FIG. 5 from the assays are provided in Table 6 and Table 7.

| Table 6. Gel lanes showing the amplicons from assay A05-216 with different omega3 DNA spike levels and 8 regular canola lines, as depicted in FIG. 4. | | | | | |
|---|---|---|---|---|---|
| Lane | Assay | Sample | Lane | Assay | Sample |
| 1 | DNA ladder | DNA ladder | 12 | A05-216 | 0.025% spike |
| 2 | A05-216 | AV Jade (neg control) | 13 | A05-216 | NX0026 |
| 3 | A05-216 | NTC | 14 | A05-216 | NX0331 |
| 4 | A05-216 | 50% DHA spike | 15 | A05-216 | NX0953 |
| 5 | A05-216 | 50% DHA spike | 16 | A05-216 | NX0980 |
| 6 | A05-216 | 10% spike | 17 | A05-216 | NX1012 |
| 7 | A05-216 | 1% spike | 18 | A05-216 | NX1302 |
| 8 | A05-216 | 0.1% spike | 19 | A05-216 | NX1306 |
| 9 | A05-216 | 0.05% spike | 20 | A05-216 | Canola 0306-B4 |
| 10 | A05-216 | 0.05% spike | 21 | A05-216 | 100% DHA |
| 11 | A05-216 | 0.05% spike | 22 | DNA ladder | DNA ladder |

| Table 7. Gel lanes showing the amplicons from assay A05-216 with 16 replicates of event positive 0.05% spike sample, as depicted in FIG. 5. | | | | | |
|---|---|---|---|---|---|
| Lane | Assay | Sample | Lane | Assay | Sample |
| 1 | DNA ladder | DNA ladder | 13 | A05-216 | Event positive 0.05% |
| 2 | A05-216 | Event Positive 0.1% | 14 | A05-216 | Event positive 0.05% |
| 3 | A05-216 | Event Positive 0.1% | 15 | A05-216 | Event positive 0.05% |
| 4 | A05-216 | Event positive 0.05% | 16 | A05-216 | Event positive 0.05% |
| 5 | A05-216 | Event positive 0.05% | 17 | A05-216 | Event positive 0.05% |
| 6 | A05-216 | Event positive 0.05% | 18 | A05-216 | Event positive 0.05% |
| 7 | A05-216 | Event positive 0.05% | 19 | A05-216 | Event positive 0.05% |
| 8 | A05-216 | Event positive 0.05% | 20 | A05-216 | Event positive 0.1% |
| 9 | A05-216 | Event positive 0.05% | 21 | A05-216 | Event positive 0.1% |
| 10 | A05-216 | Event positive 0.05% | 22 | A05-216 | Event positive 0.025% |
| 11 | A05-216 | Event positive 0.05% | 23 | A05-216 | AV Jade (DHA negative) |
| 12 | A05-216 | Event positive 0.05% | 24 | A05-216 | NTC |

### Specificity

### Specificity of the qualitative event-specific Assay and Reference Gene Assay HMG in various events among same species (Canola)

The qualitative event-specific Assay (A05-216) and reference gene assay HMG206 were tested with six different commercial canola GMO events ordered from AOCS. The results showed assay A05-216 (FIG. 6) did not have any amplicons from six commercial canola events. The reference gene assay HMG206 amplified the expected amplicons from the six commercial canola events as expected. The results demonstrated the assay A05-216 developed is specific for NS-50027-4 event in canola.

| Table 8. Gel lanes showing the amplicons from assay A05-216 with 6 different commercial canola GMO events ordered from AOCS, as depicted in FIG. 6. | | | | | |
|---|---|---|---|---|---|
| Lane | Assay | Sample | Lane | Assay | Sample |
| 1 | DNA ladder | DNA ladder | 11 | HMG206 | Canola event T45 |
| 2 | A05-216 | Event Positive control | 12 | HMG206 | Canola event Rf1 |
| 3 | A05-216 | 100% DHA Positive | 13 | HMG206 | Canola event Rf2 |
| 4 | A05-216 | AV Jade (DHA negative) | 14 | HMG206 | Canola event Rf3 |
| 5 | A05-216 | Canola event T45 | 15 | HMG206 | Canola event Ms8 |
| 6 | A05-216 | Canola event Rf1 | 16 | HMG206 | Canola event Ms 1 |
| 7 | A05-216 | Canola event Rf2 | 17 | HMG206 | AV Jade HMG positive |
| 8 | A05-216 | Canola event Rf3 | 18 | HMG206 | NTC |
| 9 | A05-216 | Canola event Ms8 | 19 | DNA ladder | DNA ladder |
| 10 | A05-216 | Canola event Ms1 | | | |

### Specificity of the qualitative event-specific Assay in various events across Brassica napus and other different species:

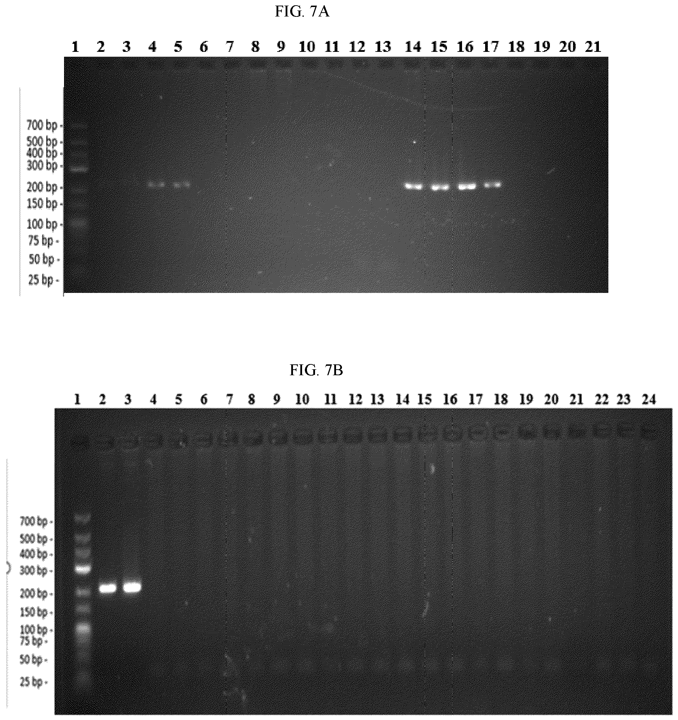

The qualitative event-specific Assay A05-216 were tested with twenty-seven CRM materials ordered from AOCS including twenty-five different commercial GMO events. The results showed assay A05-216 did not have any amplicons from all these materials, including in eight canola CRM (seven GMO events and one non-GMO canola (FIG. 7A), seven soybean events, seven maize events, four cotton events and one non-GMO cotton (FIG. 7B). The DHA canola positive controls amplified the expected amplicons in the same experiment. The results demonstrated the assay A05-216 provided herein is event specific for only canola NS-B50027-4 event.

| Table 9. Gel lanes showing the amplicons from assay A05-216 with eight canola Certified Materials (CRM; 7 GM and 1 regular canola materials), as shown in FIG. 7A. | | | | | |
|---|---|---|---|---|---|
| Lane | Assay | Sample | Lane | Assay | Sample |
| 1 | DNA ladder | DNA ladder | 12 | A05-216 | Canola event Ms 1 |
| 2 | A05-216 | 0.025% Event Positive | 13 | A05-216 | Canola event Rf3 |
| 3 | A05-216 | 0.025% Event Positive | 14 | A05-216 | Event Positive control |
| 4 | A05-216 | 0.05% Event Positive | 15 | A05-216 | Event Positive control |
| 5 | A05-216 | 0.05% Event Positive | 16 | A05-216 | 50% Event Positive |
| 6 | A05-216 | Non-Modified canola 0306-B4 | 17 | A05-216 | 10% Event Positive |
| 7 | A05-216 | Canola event Topas19/2 | 18 | A05-216 | AV Jade (DHA negative) |
| 8 | A05-216 | Canola event T45 | 19 | A05-216 | AV Jade (DHA negative) |
| 9 | A05-216 | Canola event Rf2 | 20 | A05-216 | NTC |
| 10 | A05-216 | Canola event Rf1 | 21 | A05-216 | NTC |
| 11 | A05-216 | Canola event Ms8 | | | |

| Table 10. Gel lanes showing the amplicons from assay A05-216 with seven soybean, seven maize and five cotton Certified Reference Materials (4 GM and 1 regular cotton materials), as shown in FIG. 7B. | | | | | |
|---|---|---|---|---|---|
| Lane | Assay | Sample | Lane | Assay | Sample |
| 1 | DNA ladder | DNA ladder | 13 | A05-216 | Maize event MON89034 |
| 2 | A05-216 | Event Positive control | 14 | A05-216 | Maize event MIR604 |
| 3 | A05-216 | 100% DHA Positive | 15 | A05-216 | Maize event MON88017 |
| 4 | A05-216 | AV Jade (DHA negative) | 16 | A05-216 | Maize event MON87427 |
| 5 | A05-216 | Soybean event MON89788 | 17 | A05-216 | Maize event MON 87460 |
| 6 | A05-216 | Soybean event MON87769 | 18 | A05-216 | Maize event T25 |
| 7 | A05-216 | Soybean event MON87708 | 19 | A05-216 | Cotton event MON15985-7 |
| 8 | A05-216 | Soybean event MON87705 | 20 | A05-216 | Cotton event MON531 |
| 9 | A05-216 | Soybean event MON87701 | 21 | A05-216 | Cotton event MON1445 |
| 10 | A05-216 | Soybean event FG72 | 22 | A05-216 | Non-GM cotton 0306-A4 |
| 11 | A05-216 | Soybean event A5547-127 | 23 | A05-216 | Cotton event GHB614 |
| 12 | A05-216 | Maize event GA21 | 24 | A05-216 | NTC |

No amplicons of the qualitative event-specific Assay A05-216 were present in non-GM canola materials. More specifically, the qualitative event-specific Assay A05-216 was also validated with eight non-GM conventional canola varieties. Seven were selected from Nuseed germplasm pool with various genetic backgrounds (FIG. 4) and one was ordered from AOCS. The results showed A05-216 assay did not amplify any amplicons from these 8 non-GM conventional canola varieties.

The event specific assay A05-216 was tested with NS-B50027-4 event line DNA at six different spike levels and sensitivity (%) was calculated based on 16 replicates at 0.05% spike level. All samples containing event NS-B50027-4 DNA from 50% to 0.05% (0.05% equals to 43.4 genome copies for NS-B50027-4, *see* Table 2) showed the expected amplicons consistently from A05-216 assay. So, the Limit of Detection (LOD) is at least 0.05% NS-B50027-4 DNA to total DNA or less than 50 genome copies.

The event specific assay A05-216 was also tested with twenty-five commercially available GM events from AOCS and eight different non-GM conventional oilseeds (*Brassica napus*) varieties. The results showed the assay can amplify the expected amplicons only from NS-B50027-4. The event specific assay A05-216 can specifically detect the DNA of DHA canola.

The HMG reference gene PCR profile was designed as internal control, all oilseed samples tested showed the expected 206-bp amplicon in the same lab setting and PCR condition.

Event-specific gel-based assay A05-216, targeting the insert in DHA canola NS-B50027-4 on chromosome A05, has been successfully developed and validated. The assay can be used for adventitious presence testing, trait purity testing, and trait introgression, and to support DHA Canola NS-B50027-4 regulatory submission and commercialization.

### Example 2. Gel electrophoresis-based A05-200 assay for qualitative detection of the transgenic event in DHA canola NS-B50027-4.

This example provides a qualitative detection method developed to determine the presence of DHA Canola (Event NS-B50027-4) in oilseeds DNA sample. As shown herein, the assay can be used for adventitious presence testing, trait purity testing, and trait introgression, and to support DHA Canola NS-B50027-4 regulatory submission and commercialization.

Event-specific gel-based assay A05-200, targeting the insert in DHA canola NS-B50027-4 on chromosome A05 has been successfully developed. The HMG reference gene PCR profile was designed as reference gene to confirm the PCR ability of DNA sample.

The Limit of Detection (LOD) of the event-specific A02 assay is determined at least 0.05% NS-B50027-4 DNA to total DNA, or less than 50 genome copies.

DNAs were extracted from the seeds using CTAB DNA extraction method, as described in Example 1.

Samples with different DHA canola DNA spikes were prepared according to the protocol, as described in Example 1.

Six DHA canola NS-B50027-4 spike levels were made as described below, the genome copies number of reference gene HMG and DHA canola is listed in Table 2.

PCR was conducted as described in Example 1. For PCR assembly: Assemble all reaction (25 µL/reaction) components (New England BioLabs (NEB) Inc., Catalog Log # M0480L) on ice, as illustrated in Table 3.

For PCR cycling profile: PCR was used for amplification with the following profile parameters, as described in Example 1, Table 4.

The canola HMG gene is used as the internal reference gene for this qualitative detection method. The Thermo Scientific^{™} GeneRuler^{™} Low Range DNA Ladder (ready-to-use) containing a mix often chromatography-purified individual DNA fragments (in base pairs: 700, 500, 400, 300, 200, 150, 100, 75, 50, 25) is used as a reference guide. *See* FIG. 1.

Agarose gel electrophoresis was conducted according to this protocol: For each sample, 10 µL PCR products, 3 µL H₂O and 1 µL loading dye (6x; Thermo Fisher Scientific #R1161) were mixed before loading, and 6 µL DNA Ladder was used. A 2% agarose gel was run at 100 Volts for 60 min. An image of the gel was captured using the Life Technology Image system.

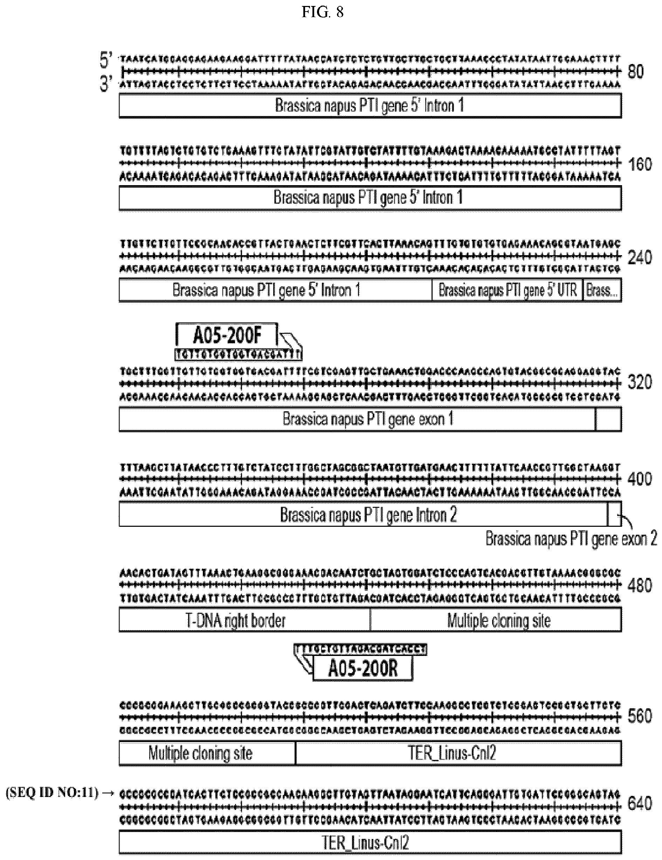

Two primer pairs were employed for this qualitative detection method. The amplicon size for HMG is 206 bp. Assay A05-200 was designed to detect the junction between T-DNA insert and genomic DNA on chromosome A05. The primer sequences, locations, and product sizes are shown in Table 11 and FIG.8. The *Brassica* HMG gene was used as the reference gene for the internal control recommended by Chinese National Standard (MARA 2031-9-2013).

| Table 11: Primer sequences for assay A05-200 and HMG | | | |
|---|---|---|---|
| Name | Sequence | Product size | Target |
| A05-200F | TGTTGTGGTGGTGACGATTT (SEQ ID NO:9) | 200 bp | Junction on Chromosome A05 (SEQ ID NO:11) |
| A05-200R | TCCACTAGCAGATTGTCGTTT (SEQ ID NO:10) | | |
| Hmg206F | TCCTTCCGTTTCCTCGCC (SEQ ID NO:5) | 206 bp | Internal reference gene HMG as control |
| Hmg206R | TTCCACGCCCTCTCCGCT (SEQ ID NO:6) | | |

Samples of 100 ng of genomic DNA template, as provided herein, were subjected to event-specific qualitative PCR (Assay A05-200 for junction in chromosome A05 and Assay HMG for Reference Gene):
- Event NS-50027-4 as positive sample for Assay A05-200;
- Receptor canola AV Jade as positive sample for Reference Gene Assay HMG; and
- Six different GM spike level samples (Table 2)

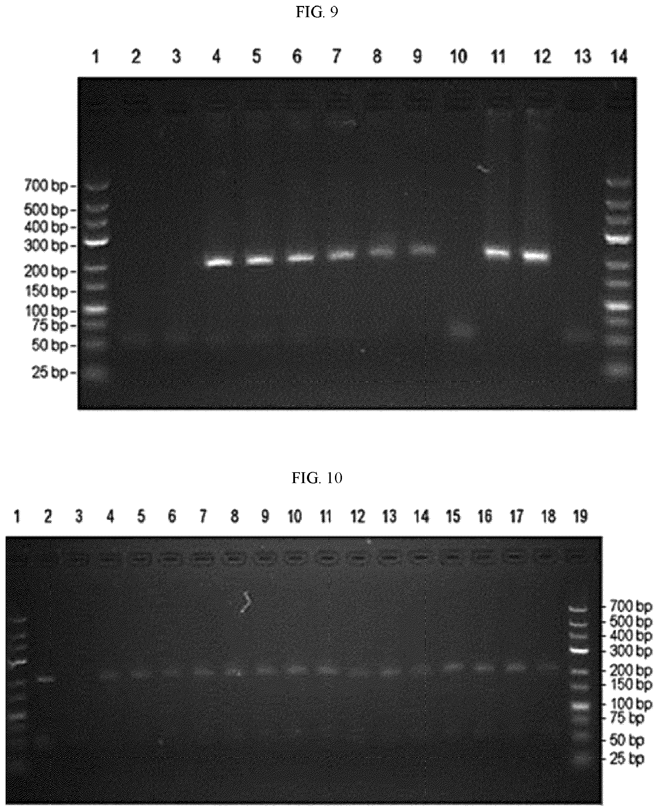

Assay A05-200 was tested along with AV Jade, NTC (no template control) and six different DHA canola NS-B50027-4 spike levels *(see* FIG. 9). In addition, the results from fifteen replicates showed both assays can consistently detect the expected amplicons at least 0.05% spike level *(see* FIG. 10). There was no amplicon from all reactions from AV Jade (negative control) for event specific assay A05-200.

The sample layout of the gel images for FIG. 9 and FIG. 10 are described in Tables 12 and 13, respectively.

| Table 12: Sample layout for assay A05-200 with different DHA GMO-canola NS-B50027-4 spike samples and HMG-206 reference gene as depicted in FIG. 9. | | | | | |
|---|---|---|---|---|---|
| Well | Assay | Sample | Well | Assay | Sample |
| 1 | DNA ladder | DNA ladder | 8 | A05-200 | 0.1% spike |
| 2 | A05-200 | AV Jade | 9 | A05-200 | 0.05% spike |
| 3 | A05-200 | NTC | 10 | A05-200 | 0.025% spike |
| 4 | A05-200 | 100% DHA canola | 11 | HMG206 | AV Jade |
| 5 | A05-200 | 50% spike | 12 | HMG206 | 0.05% spike |
| 6 | A05-200 | 10% spike | 13 | HMG206 | NTC |
| 7 | A05-200 | 1% spike | 14 | DNA ladder | DNA ladder |
| NTC=no template control | | | | | |

| Table 13: Sample layout for assay A05-200 with 15 replicates of event positive 0.05% spike sample as depicted in FIG. 10. | | | | | |
|---|---|---|---|---|---|
| Lane | Assay | Sample | Lane | Assay | Sample |
| 1 | DNA ladder | DNA ladder | 11 | A05-200 | Event positive 0.05% |
| 2 | A05-200 | Event Positive 0.1% | 12 | A05-200 | Event positive 0.05% |
| 3 | A05-200 | AV Jade (control) | 13 | A05-200 | Event positive 0.05% |
| 4 | A05-200 | Event positive 0.05% | 14 | A05-200 | Event positive 0.05% |
| 5 | A05-200 | Event positive 0.05% | 15 | A05-200 | Event positive 0.05% |
| 6 | A05-200 | Event positive 0.05% | 16 | A05-200 | Event positive 0.05% |
| 7 | A05-200 | Event positive 0.05% | 17 | A05-200 | Event positive 0.05% |
| 8 | A05-200 | Event positive 0.05% | 18 | A05-200 | Event positive 0.05% |
| 9 | A05-200 | Event positive 0.05% | 19 | DNA ladder | DNA ladder |
| 10 | A05-200 | Event positive 0.05% | | | |

The event specific assay A05-200 was tested with NS-B50027-4 event line DNA at six different spike levels and sensitivity (%) was calculated based on fifteen replicates. All samples containing event NS-B50027-4 DNA from 50% to 0.05% (0.05% equals 43.4 genome copies for NS-B50027-4, *see* Table 2) showed the expected amplicons consistently from A05-200 assay. The Limit of Detection (LOD) is at least 0.05% NS-B50027-4 DNA to total DNA or less than 50 genome copies. No amplicon was detected from AV Jade (a negative control) for event-specific assay A05-200.

Event-specific gel-based assay A05-200, targeting the insert in DHA canola NS-B50027-4 on chromosome A05 has been successfully developed. The assay may be used for adventitious presence testing, trait purity testing, and trait introgression, and to support Nuseed DHA Canola NS-B50027-4 regulatory submission and commercialization.

The specificity of the event-specific A05-200 assay, as discussed above, was further validated by testing twenty-five commercially available GM events (including seven canola events, seven soybean events, seven maize events and four cotton events) from AOCS and seven different non-GM conventional oilseeds varieties. The validation data showed that the assays can detect positive results only from DHA samples. All CRM materials and conventional oilseeds varieties showed negative results for Event NS-B50027-4.

To carry out the validation assays, DNA were extracted from seeds using CTAB DNA extraction, as described in Example 1.

Samples of 100 ng genomic DNA template, as described herein, were subjected to event-specific qualitative PCR (assay A05-200 for junction in chromosome A05 and assay HMG for Reference Gene):
- Event NS-50027-4 as positive sample for Assay A05-200;
- Receptor canola AV Jade as positive sample for Reference Gene Assay HMG;
- Non-GM conventional canola varieties selected from Nuseed germplasm pool: NX0026, NX0331, NX0953, NX0980, NX1012, NX1302, NX1306;
- Certified Non-Modified Reference Materials (Leaf DNA) purchased from American Oil Chemists' Society (AOCS): Canola 0306-B4, Cotton 0306-A4;
- Certified GM Reference Materials (Leaf DNA or seeds powder) purchased from AOCS;
- Seven Canola Events: Topas19/2, T45, Rf2, Rf1, Ms8, Ms1, Rf3;
- Seven Soybean Events: MON89788, MON87769, MON87708, MON87705, MON87701, FG72, A5547-127;
- Seven Maize Events: GA21, MON89034, MIR604, MON88017, MON87427, MON87460, T25;
- Four Cotton Events: MON15985-7, MON531, MON1445, GHB614.

The parameters for PCR assembly, PCR cycling profile and primers, as used herein, were identical with those described in Tables 3, 4 and 5, respectively

For each sample, 10 µL PCR products, 3 µL H₂O, and 1 µL loading dye (6x; Thermo Fisher Scientific #R1161) were mixed before loading, and 6 µL DNA Ladder was used. A 2% agarose gel was run at 100 Volts for 60 min. The gel was then photographed using a Life Technology Image system.

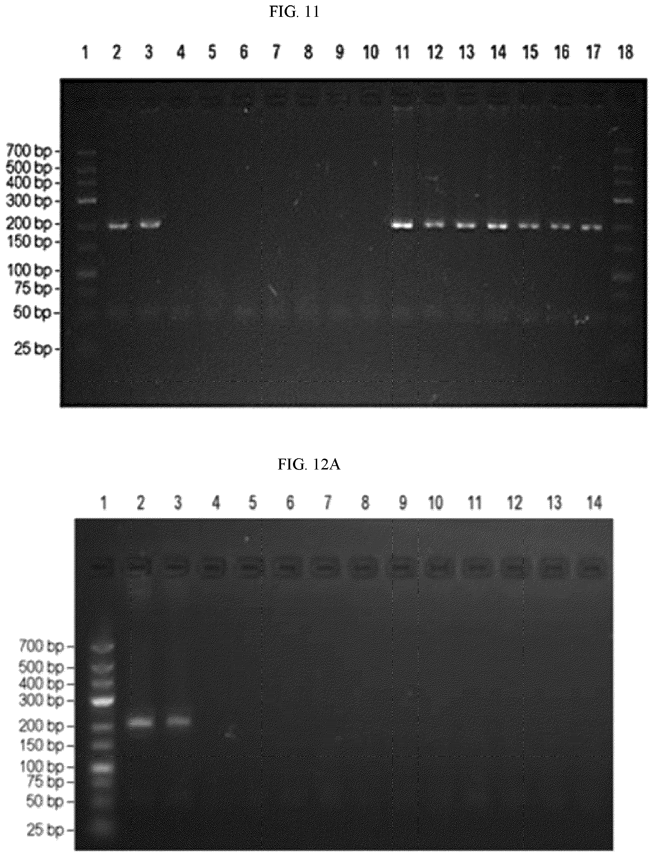

Assays A05-200 and HMG were validated with six different commercial canola GMO events ordered from AOCS. The results showed assay A05-200 *(see* FIG. 11) did not have any amplicons from six commercial canola events. The control gene assay HMG amplified the expected amplicons from these six commercial canola events as expected. The results demonstrated that assay A05-200 developed at Nuseed was event-specific for canola. The sample layout for FIG. 11 is described in Table 14.

| Table 14: Sample layout for validation of assay A05-200 plus HMG 206 with six CRM canola events as illustrated in FIG. 11. | | | | | |
|---|---|---|---|---|---|
| Well | Assay | Sample | Well | Assay | Sample |
| 1 | DNA ladder | DNA ladder | 10 | A05-200 | Canola event Ms 1 |
| 2 | A05-200 | DHA Positive | 11 | A05-200 | DHA Positive |
| 3 | A05-200 | DHA Positive | 12 | HMG206 | Canola event Topas 19/2 |
| 4 | A05-200 | AV Jade (DHA negative) | 13 | HMG206 | Canola event T45 |
| 5 | A05-200 | Canola event Topas 19/2 | 14 | HMG206 | Canola event Rf2 |
| 6 | A05-200 | Canola event T45 | 15 | HMG206 | Canola event Rf1 |
| 7 | A05-200 | Canola event Rf2 | 16 | HMG206 | Canola event Ms8 |
| 8 | A05-200 | Canola event Rf1 | 17 | HMG206 | Canola event Ms 1 |
| 9 | A05-200 | Canola event Ms8 | 18 | DNA ladder | DNA ladder |

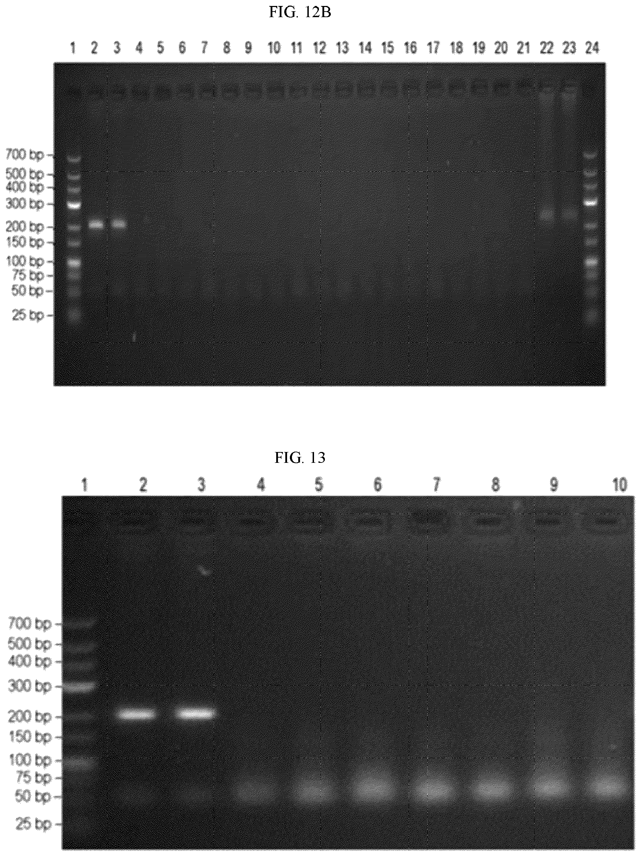

Assays A05-200 and HMG were further validated with all twenty-five different commercial GMO events ordered from AOCS. The results showed assay A05-200 did not have any amplicons from all these events, including in seven canola events, seven soybean events, seven maize events, and four cotton events. The DHA canola-positive controls amplified the expected amplicons in the same experiment *(see* FIG. 12A and FIG. 12B for assay A05-200). The results demonstrated that assay A05-200 developed at Nuseed was event-specific for only canola NS-B50027-4 event. The sample layout for FIG. 12A and FIG. 12B is described below in Tables 15A and 15B, respectively.

| Table 15A: Sample layout for validation of assay A05-200 with 25 Certified GM Reference Materials as illustrated in FIG. 12A. | | | | | |
|---|---|---|---|---|---|
| Well | Assay | Sample | Well | Assay | Sample |
| 1 | DNA ladder | DNA ladder | 8 | A05-200 | Canola event Rf2 |
| 2 | A05-200 | Event Positive | 9 | A05-200 | Canola event Rf1 |
| 3 | A05-200 | Event Positive | 10 | A05-200 | Canola event Ms8 |
| 4 | A05-200 | AV Jade (DHA negative) | 11 | A05-200 | Canola event Ms 1 |
| 5 | A05-200 | Non-GMO canola 0306-B4 | 12 | A05-200 | Canola event Rf3 |
| 6 | A05-200 | Canola event Topas19/2 | 13 | A05-200 | Soybean event MON89788 |
| 7 | A05-200 | Canola event T45 | 14 | A05-200 | Soybean event MON87769 |

| Table 15B: Sample layout for validation of assay A05-200 with 25 Certified GM Reference Materials as illustrated in FIG. 12B. | | | | | |
|---|---|---|---|---|---|
| Well | Assay | Sample | Well | Assay | Sample |
| 1 | DNA ladder | DNA ladder | 13 | A05-200 | Maize event MON88017 |
| 2 | A05-200 | Event Positive | 14 | A05-200 | Maize event MON87427 |
| 3 | A05-200 | Event Positive | 15 | A05-200 | Maize event MON 87460 |
| 4 | A05-200 | AV Jade (DHA negative) | 16 | A05-200 | Maize event T25 |
| 5 | A05-200 | Soybean event MON87708 | 17 | A05-200 | Cotton event MON15985-7 |
| 6 | A05-200 | Soybean event MON87705 | 18 | A05-200 | Cotton event MON531 |
| 7 | A05-200 | Soybean event MON87701 | 19 | A05-200 | Cotton event MON1445 |
| 8 | A05-200 | Soybean event FG72 | 20 | A05-200 | Non-Modified cotton 0306-A4 |
| 9 | A05-200 | Soybean event A5547-127 | 21 | A05-200 | Cotton event GHB614 |
| 10 | A05-200 | Maize event GA21 | 22 | HMG206 | Event Positive |
| 11 | A05-200 | Maize event MON89034 | 23 | HMG206 | Event Negative |
| 12 | A05-200 | Maize event MIR604 | 24 | DNA ladder | DNA ladder |

Assay A05-200 was further validated with seven non-GM conventional canola varieties selected from Nuseed germplasm pool with various genetic backgrounds. The results showed that assay A05-200 did not amplify any amplicon from these seven non-GM conventional canola varieties *(see* FIG. 13). Sample layout of FIG. 13 is described in Table 17.

| Table 17: Sample layout for validation of assay A05-200 with seven non-GM conventional canola varieties as illustrated in FIG. 13 | | | | | |
|---|---|---|---|---|---|
| Well | Assay | Sample | Well | Assay | Sample |
| 1 | DNA ladder | DNA ladder | 6 | A05-200 | NX0953 |
| 2 | A05-200 | Event Positive | 7 | A05-200 | NX0980 |
| 3 | A05-200 | Event Positive | 8 | A05-200 | NX1012 |
| 4 | A05-200 | NX0026 | 9 | A05-200 | NX1302 |
| 5 | A05-200 | NX0331 | 10 | A05-200 | NX1306 |

The specificity of the A05-200 assay was validated from testing twenty-five commercially available GM events (including seven canola events, seven soybean events, seven maize events and four cotton events) from AOCS and eight different non-GM conventional oilseeds varieties (one from AOCS and seven from Nuseed germplasm pool). Accordingly, the event-specific gel-based assay, A05-200, targeting the insert in DHA canola NS-B50027-4 on chromosome A05 was successfully developed and validated. The assay can be used for adventitious presence testing, trait purity testing, and trait introgression, and to support regulatory submission, stewardship, and commercialization.

The twenty-seven Certified Reference Materials (CRM) from American Oil Chemists' Society (AOCS) employed and tested in the above study are listed in Table A:

| Table A: List of CRM | |
|---|---|
| Certified Reference materials from AOCS (American Oil Chemists' Society) | Sample ID |
| Canola Events: | |
| 0306B4-Canola Bayer CropScience Non-Modified Leaf DNA | AOCS-1 |
| 0711D3-Canola Bayer CropScience Event Topas 19/2 Leaf DNA | AOCS-2 |
| 0208A5-Canola Bayer CropScience Event T45 Genomic DNA Leaf Tissue | AOCS-3 |
| 0711C2-Canola Bayer CropScience Event Rf2 Leaf DNA | AOCS-4 |
| 0711B2-Canola Bayer CropScience Event Rf1 Leaf DNA | AOCS-5 |
| 0306F6-Canola Bayer CropScience Event Ms8 Leaf DNA | AOCS-6 |
| 0711A3-Canola Bayer CropScience Event Ms1 Leaf DNA | AOCS-7 |
| 0306G5-Canola Bayer Crop Science Event Rf3 Leaf DNA | AOCS-8 |

| Soybean Events: | |
|---|---|
| 0906B-Soybean Monsanto Company Event MON89788 | AOCS-9 |
| 0809B-Soybean Monsanto Company Event MON87769 | AOCS-10 |
| 0311A-Soybean Monsanto Company Event MON87708 | AOCS-11 |
| 0210A-Soybean Monsanto Company Event MON87705 | AOCS-12 |
| 0809A-Soybean Monsanto Company Event MON87701 | AOCS-13 |
| 0610A3-Soybean Bayer CropScience Event FG72 Leaf DNA | AOCS-14 |
| 0707C6-Soybean Bayer CropScience Event A5547-127 Leaf DNA | AOCS-15 |

| Table A: List of CRM | |
|---|---|
| Certified Reference materials from AOCS (American Oil Chemists' Society) | Sample ID |
| Maize Events: | |
| 0407B-Maize Syngenta Event GA21 | AOCS-16 |
| 0906E-Maize Monsanto Company Event MON89034 | AOCS-17 |
| 0607A2-Maize Syngenta Event MIR604 | AOCS-18 |
| 0406D-Maize Monsanto Company Event MON88017 | AOCS-19 |
| 0512A-Maize Monsanto Company Event MON87427 | AOCS-20 |
| 0709A-Maize Monsanto Company Event MON87460 | AOCS-21 |
| 0306H9-Maize Bayer CropScience Event T25 DNA | AOCS-22 |

| Cotton Events: | |
|---|---|
| 0804D-Cotton Monsanto Company Event MON15985-7 | AOCS-23 |
| 0804C-Cotton Monsanto Company Event MON531 | AOCS-24 |
| 0804B-Cotton Monsanto Company Event MON1445 | AOCS-25 |
| 0306A3-Cotton Bayer CropScience Non-Modified Leaf DNA | AOCS-26 |
| 1108A5-Cotton Bayer CropScience Event GHB 614 Leaf DNA | AOCS-27 |

It is to be understood that the disclosure has been described in conjunction with the above embodiments, that the preceding description and examples are intended to illustrate and not limit the scope of the disclosure. Other aspects, advantages, and modifications within the scope of the disclosure will be apparent to those skilled in the art to which the disclosure pertains.

## Claims

1. A qualitative gel-based method of identifying the presence of a DHA canola NS-B50027-4 event or progeny thereof in a biological sample, said method comprising detecting a two event-specific detection method that comprises
(a) extracting a DNA sample from said biological sample;
(b) providing a first DNA primer pair combination, wherein said first DNA primer pair combination is from nucleotides as set forth in SEQ ID NO: 1 (A05-216F) and SEQ ID NO:2 (A05-216R);
(c) providing a second DNA primer pair combination, wherein said second DNA primer pair combination is from nucleotides as set forth in SEQ ID NO:3 (A02-258F) and SEQ ID NO:4 (A02-258R);
(d) providing DNA amplification reaction conditions; and
(e) performing two separate DNA amplification reactions with said first (as in step (b)) and second (as in step (c)) DNA primer pair combinations, thereby producing an amplicon pair consisting of a 216-bp amplicon and a 258-bp amplicon, respectively;
wherein the presence of said amplicon pair identifies the presence of the DHA canola NS- B50027-4 event or progeny thereof in the biological sample.

2. The method of claim 1, wherein the first DNA primer pair combination targets a transgenic junction region on chromosome A05.

3. The method of claim 1, wherein the second DNA primer pair combination targets a transgenic junction region on chromosome A02.

4. The method of claim 1, wherein said method is used for adventitious presence testing, low level presence testing, trait purity testing, trait introgression testing, or supporting plant stewardship of the DHA canola designated NS-B50027-4.

5. The method of claim 1, where said qualitative detection uses a polymerase chain reaction (PCR).

## Patentansprüche

1. Qualitatives Verfahren auf Gelbasis zum Identifizieren des Vorliegens eines DHA-Raps-NS-B50027-4-Event oder von Nachkommenschaft davon in einer biologischen Probe, wobei das Verfahren Finden eines für zwei Events spezifischen Nachweisverfahrens umfasst, das Folgendes umfasst:
(a) Extrahieren einer DNA-Probe aus der biologischen Probe;
(b) Bereitstellen einer ersten DNA-Primerpaar-Kombination, wobei die erste DNA-Primerpaar-Kombination aus Nukleotiden gemäß SEQ ID NO:1 (A05-216F) und SEQ ID NO:2 (A05-216R) besteht;
(c) Bereitstellen einer zweiten DNA-Primerpaar-Kombination, wobei die zweite DNA-Primerpaar-Kombination aus Nukleotiden gemäß SEQ ID NO: 3 (A02-258F) und SEQ ID NO: 4 (A02-258R) besteht;
(d) Bereitstellen von DNA-Amplifikationsreaktionsbedingungen; und
(e) Durchführen von zwei getrennten DNA-Amplifikations-reaktionen mit der ersten (gemäß Schritt (b)) bzw. der zweiten (gemäß Schritt (c)) DNA-Primerpaar-Kombination, wodurch ein Amplifikatpaar erzeugt wird, das aus einem 216-bp-Amplifikat bzw. einem 258-bp-Amplifikat besteht;
wobei durch das Vorliegen des Amplifikatpaars das Vorliegen des DHA-Raps-NS-B50027-4-Event oder von Nachkommenschaft davon in der biologischen Probe identifiziert wird.

2. Verfahren nach Anspruch 1, wobei die erste DNA-Primerpaar-Kombination auf eine transgene Verbindungsregion auf Chromosom A05 zielt.

3. Verfahren nach Anspruch 1, wobei die zweite DNA-Primerpaar-Kombination auf eine transgene Verbindungsregion auf Chromosom A02 zielt.

4. Verfahren nach Anspruch 1, wobei das Verfahren zum Testen auf zufälliges Vorliegen, Testen auf Vorliegen auf niedrigem Niveau, Testen auf Merkmalsreinheit, Testen auf Merkmalsintrogression oder Unterstützen der Pflanzenbetreuung des DHA-Raps mit der Bezeichnung NS-B50027-4 verwendet wird.

5. Verfahren nach Anspruch 1, bei dem zum qualitativen Nachweis eine Polymerase-Kettenreaktion (PCR= polymerase chain reaction) genutzt wird.

## Revendications

1. Procédé à base de gel qualitatif d'identification de la présence d'un événement NS-B50027-4 de canola DHA ou d'une descendance correspondante dans un échantillon biologique, ledit procédé comprenant la détection d'un procédé de détection spécifique à deux événements qui comprend
(a) l'extraction d'un échantillon d'ADN ou dudit échantillon biologique ;
(b) la fourniture d'une première combinaison de paires d'amorces d'ADN, ladite première combinaison de paires d'amorces d'ADN provenant de nucléotides comme indiqués dans la SEQ ID NO: 1 (A05-216F) et la SEQ ID NO: 2 (A05-216R) ;
(c) la fourniture d'une deuxième combinaison de paires d'amorces d'ADN, ladite deuxième combinaison de paires d'amorces d'ADN provenant de nucléotides comme indiqués dans la SEQ ID NO: 3 (A02-258F) et la SEQ ID NO: 4 (A02-258R) ;
(d) la fourniture de conditions de réaction d'amplification d'ADN ; et
(e) la réalisation de deux réactions d'amplification d'ADN distinctes avec lesdites première (comme dans l'étape (b)) et deuxième (comme dans l'étape (c)) combinaisons de paires d'amorces d'ADN, produisant ainsi une paire d'amplicons constituée respectivement d'un amplicon de 216 pb et d'un amplicon de 258 pb ;
la présence de ladite paire d'amplicons identifiant la présence de l'événement NS-B50027-4 de canola DHA ou d'une descendance correspondante dans l'échantillon biologique.

2. Procédé selon la revendication 1, la première combinaison de paires d'amorces d'ADN ciblant une région de jonction transgénique sur le chromosome A05.

3. Procédé selon la revendication 1, la deuxième combinaison de paires d'amorces d'ADN ciblant une région de jonction transgénique sur le chromosome A02.

4. Procédé selon la revendication 1, ledit procédé étant utilisé pour un test de présence accidentelle, un test de présence à faible niveau, un test de pureté de trait, un test d'introgression de trait, ou le soutien de la gestion d'un végétal du canola DHA désigné NS-B50027-4.

5. Procédé selon la revendication 1, où ladite détection qualitative utilise une réaction en chaîne par polymérase (PCR).
